(19)

**Europäisches Patentamt**
**European Patent Office**
**Office européen des brevets**

(11)  **EP 1 351 887 B1**

(12)  **EUROPEAN PATENT SPECIFICATION**

(45) Date of publication and mention
of the grant of the patent:
**01.10.2008  Bulletin 2008/40**

(51) Int Cl.:
*C01F 7/76* ^(2006.01)    *A61K 33/08* ^(2006.01)

(21) Application number: **01993583.2**

(86) International application number:
**PCT/EP2001/012849**

(22) Date of filing: **07.11.2001**

(87) International publication number:
**WO 2002/038500 (16.05.2002 Gazette 2002/20)**

(54) **PROCESS FOR THE REHYDRATION OF MAGALDRATE POWDER**

VERFAHREN ZUR REHYDRATISIERUNG VON MAGALDRAT-PULVER

R HYDRATATION DE LA POUDRE DE MAGALDRATE

(84) Designated Contracting States:
**AT BE CH CY DE DK ES FI FR GB GR IE IT LI LU MC NL PT SE TR**
Designated Extension States:
**AL LT LV MK RO SI**

(30) Priority: **08.11.2000 EP 00124194**

(43) Date of publication of application:
**15.10.2003  Bulletin 2003/42**

(73) Proprietor: **Nycomed GmbH**
**78467 Konstanz (DE)**

(72) Inventors:
• **BRÜGGEMANN, Juliane**
  **78464 Konstanz (DE)**
• **TRÖSSER, Heinrich**
  **78476 Allensbach (DE)**

(56) References cited:
**EP-A- 0 154 114       EP-A- 0 178 895**
**EP-A- 0 219 740       DE-A- 4 018 628**
**US-A- 2 923 660       US-A- 5 437 873**

## Description

### Technical field

The present invention relates to the field of pharmaceutical technology and describe a novel process for the rehydration of magaldrate powder.

### Background art

Magaldrate has been used for a long time as antacid product (magaldrate is the international nonproprietary name for an antacid based on aluminum magnesium hydroxide). The commercial antacid products are marketed either as tablets or in the form of liquid suspensions. Tablets are usually produced from magaldrate powder and the liquid suspensions from magaldrate gel. If magaldrate products are sold in the form of liquid suspensions, it is difficult and costly to transport magaldrate gel over large distances to the place of production of the liquid suspensions. The suspensions can also be produced from the powder used for tablet production, but this powder cannot be completely rehydrated, and the suspension is gritty and sediments considerably. As described in US-A 2,923,660, magaldrate is usually produced by adding a magnesium salt solution to a strongly alkaline alkali metal aluminate solution. The freshly precipitated substance is In the form of a gel or In colloidal form. If the form is dried to produce a powder, the colloidal properties which were present with the freshly precipitated gel are destroyed. If the powder is put into water it cannot be completely converted back Into a hydrated or gel form. To eliminate this defect, DE 2749789 proposes adding a soluble organic colloid to the magaldrate gel. Subsequent drying is said to result in a rehydratable magaldrate powder which is converted into the hydrated form again after mixing with water.

### Description of the invention

The object of the present invention is to provide a simple process for the rehydration of magaldrate powder, in particular a process which requires no additions of excipients. It has now been found, surprisingly, that magaldrate can be obtained in hydrated form or gel form from magaldrate powder by grinding of aqueous suspensions of magaldrate powder. An addition of organic or inorganic colloids Is unnecessary In this connection. The preparations obtained after grinding of aqueous suspensions of magaldrate powder have advantageous properties for their further processing to commercial suspensions in relation to sedimentation behavior, particle distribution and flow behavior, which are comparable with those of freshly precipitated magaldrate gels. The preparations obtained in this way can be further processed without difficulty to commercial liquid suspensions. The elaborate and costly transport of freshly precipitated magaldrate gels to the place of further processing to commercial liquid suspensions can thus be omitted.

The invention therefore relates to a process for the production of a rehydrated magaldrate preparation, from magaldrate powder comprising the step of grinding of an aqueous suspension of magaldrate powder in a suitable mill.

The aqueous suspensions of magaldrate powder employed according to the invention contain 10 to 40%, preferably 18 to 22%, (w/w) magaldrate powder. Such suspensions can be obtained, for example, by adding magaldrate powder to water with stirring. If desired, it is also possible to add a preservative such as, for example, silver sulfate for the later commercial product. It is possible to employ according to the invention commercial magaldrate powder (e.g. produced as described at the outset) of variable quality. The grinding of the aqueous suspension of magaldrate powder takes place according to the invention in suitable mills, by Ultraturrax or by ultrasound. Mills suitable accordIng to the Invention are, in particular, annular gap bead mills. Annular gap bead mills are known to the skilled worker. The speed of rotation of the rotor, the width of the grinding gap, the level of the beads and the diameter of the grinding beads, and the flow rate of the suspension, can be used to influence the result of grinding.

The rehydrated magaldrate preparations obtained after grinding can then be further processed to commercial liquid suspensions. It is possible to add for this purpose for example other additives such as flavorings (e.g. maltol as flavor enhancer and aromatizers), flocculation-preventing agents, thickeners (e.g. cellulose), preservatives (e.g. silver sulfate and chlorhexidine gluconate) and sweeteners (e.g. sodium cyclamate). It has been observed that the rehydrated magaldrate preparations obtained by the process of the invention are stable on storage and, even after a prolonged period, neither flocculation nor association is to be observed.

The invention therefore further relates to a process for the production of liquid suspensions comprising magaldrate for use as pharmaceutical comprising the steps of

a) producing a rehydrated magaldrate preparation from magaldrate powder by grinding of an aqueous suspension of magaldrate powder in a suitable mill
b) mixing of the rehydrated magaldrate preparation obtained under a) with at least one additive or a suitable solution of an additive, where the additive is selected from the group of flavorings, flocculation-preventing agents, thickeners, preservatives and sweeteners.

## Examples

## Production of rehydrated preparations from magaldrate powder

Magaldrate powder of the following quality was employed:

Magaldrate R = magaldrate powder (USP), micronized;
Magaldrate LD = magaldrate powder (USP), ground;
Magaldrate HD = magaldrate powder (USP), ground.

Magaldrate LD and HD differ In the apparent density. Magaldrate R, LD and HD are obtainable, for example, from Reheis, Dublin, Ireland.

## Analytical methods

### 1) Measurement of the viscosity of the rehydrated preparations of the invention

All the measurements were carried out in an AR 1000 rheometer from TA Instruments.

Flow test 585 $s^{-1}$
Test duration 5 minutes
Test temperature 20°C
Evaluation at 585 $s^{-1}$
Measurement system: plate/cone 4 cm 2°

### 2) Measurement of the particle distribution of the rehydrated preparations of the Invention

Method: SYMTATHEC HELOS particle size analysis

### Example 1 Magaldrate R about 20% MS12

Grinding in an annular gap bead mill CoBall MS 12 mill

Motor 3 KW; grinding gap 6.5 mm; sieve 0.3 mm, separation gap 0.05 mm
Beads of zirconium oxide 0.8 mm, bead filling 60%
Circumferential speed: 13 m/s
Amperage: 2A
Product temperature in: 23°C; product temperature out: 19.5-22°C

Magaldrate powder 800 g (magaldrate R) Is dispersed with 4200 g of demineralized water. The concentration of anhydrous magaldrate is about 14% (w/w). The disperson is conveyed into the mill by means of a pump and is ground.

## Viscosities at various flow rates:

| 7 kg/h | 92 mPa.s |
|---|---|
| 10 kg/h | 69 mPa.s |
| 20 kg/h | 48 mPa.s |

## Particle distribution before grinding and with 10 kg throughput:

| Sample | X10 | X50 | X90 | X98 |
|---|---|---|---|---|
| Dispersion | 1.78 m | 6.18 $\mu$m | 13.72 $\mu$m | 19.56 $\mu$m |
| 10 kg/h | 1.52 $\mu$m | 3.71 $\mu$m | 8.49 $\mu$m | 13.39 $\mu$m |

**Example 2 Magaldrate HD about 20% MS12**

Grinding in an annular gap bead mill CoBall MS 12 mill
Motor 3 KW; grinding gap 6.5 mm; sieve 0.3 mm, separation gap 0.05 mm
Beads of zirconium silicate 0.8 mm-1.0 mm, bead filling 60%
Circumferential speed: 13 m/s
Amperage: 2A
Product temperature In: 23°C; product temperature out: 25°C
Throughput: 12 kg/h

Magaldrate powder 890 g of HD is dispersed with 3120 g of demineralized water. The concentration of anhydrous magaldrate is about 19% (w/w). The disperson is conveyed into the mill by means of a pump and is ground.

Viscosity: 159 mPa.s

Particles before grinding:   x  50 =   19.32 x  98 =  85.25

After grinding:              x  50 =   3.41 x  98 =  16.44

**Example 3 Magaldrate LD about 20% MS12**

Grinding in an annular gap bead mill CoBall MS 12 mill
Motor 3 KW; grinding gap 6.5 mm; sieve 0.3 mm, separation gap 0.05 mm
Beads of zirconium silicate 0.8 mm-1.0 mm, bead filling 60%
Circumferential speed: 13 m/s
Amperage: 2A
Product temperature in: 23°C; product temperature out: 25°C
Throughput: 12 kg/h

Magaldrate powder 890 g of LD is dispersed with 3120 g of demineralized water. The concentration of anhydrous magaldrate is about 19% (w/w). The disperson is conveyed into the mill by means of a pump and is ground.

Viscosity: 195 mPa.s

Particles before grinding:  x  50 =  19.32 x  98 =  85.25

After grinding:             x  50 =   2.95 x  98 =  17.25

### Example 4 Magaldrate R about 20% MS32

Grinding in an annular gap bead mill CoBall MS 32 mill
Motor 30 KW; grinding gap 7 mm; sieve 0.2 mm, separation gap 0.1 mm
Beads of zirconium silicate 0.8 mm-1.0 mm,
Bead filling 70% throughput 78 kg/h
Bead filling 60% throughput 105 kg/h
Circumferential speed: 13 m/s
Amperage: 22A
Product temperature in: 24.8°; product temperature out: 20.8°C

Magaldrate powder R 23.9 kg are dispersed with 76.1 kg of demineralized water. The concentration of anhydrous magaldrate is about 20% (w/w). To preserve the crude gel, 1.65 g of silver sulfate are added. The dispersion is conveyed into the mill by means of a pump and is ground.

Particle distribution:

| Throughput | X10 | X50 | X90 | X98 |
|---|---|---|---|---|
| 78 kg/h | 1.34 μm | 3.12 μm | 6.51 μm | 9.42 μm |
| 105 kg/h | 1.43 μm | 3.31 μm | 6.77 μm | 9.72 μm |

Viscosity of the preparation obtained with a throughput of 105 kg/h (60% bead filling/105 kg throughput) =118 mPa.s

### Example 5 Magaldrate R/magaldrate LD 2:1 about 20% MS32

Grinding in an annular gap bead mill CoBall MS 32 mill
Motor 22 KW; grinding gap 7 mm; sieve 0.2 mm, separation gap 0.1 mm
Beads of zirconium silicate 0.8 mm-1.0 mm,
Bead filling 60%; throughput see table
Circumferential speed: 12.6 m/s
Amperage: 25A
Product temperature in: 21°; product temperature out: 20°C

Magaldrate powder R 24 kg and magaldrate powder LD 12 kg are dispersed with 114 kg of demineralized water. The concentration of anhydrous magaldrate is about 20% (w/w). To preserve the crude gel, 2.175 g of silver sulfate are added. The dispersion is conveyed into the mill by means of a pump (NL 15A) and is ground.

| Sample | Viscosity mPa.s | X10(μm) | X50 (μm) | X90 (μm) | X100 (μm) |
|---|---|---|---|---|---|
| Un-ground |  | 2.1 | 5.6 | 21.2 | 51.5 |
| 130 kg/h | 129 | 1.4 | 3.4 | 7.3 | 18 |
| 180 kg/h | 105 | 1.5 | 3.5 | 7.6 | 21.5 |
| 120 kg/h | 97 | 1.5 | 3.5 | 7.8 | 21.5 |
| 200 kg/h | 66 | 1.6 | 3.6 | 8.5 | 25.5 |

**Example 6 Magaldrate R/magaldrate LD 1:1 about 20% MS32**

Grinding in an annular gap bead mill CoBall MS 32 mill
Motor 22 KW; grinding gap 7 mm; sieve 0.2 mm, separation gap 0.1 mm
Beads of zirconium silicate 0.8 mm-1.0 mm,
Bead filling 60%; throughput 240 kg/h
Circumferential speed: 12.6 m/s
Amperage: 25A
Product temperature In: 21°; product temperature out: 22°C
Feed pressure: 0 bar

Magaldrate powder R 36 kg and magaldrate powder LD 36 kg are dispersed with 228 kg of demineralized water. The concentration of anhydrous magaldrate is about 20% (w/w). To preserve the crude gel, 5.46 g of silver sulfate are added. The dispersion is conveyed Into the mill by means of a pump (NL 15A) and is ground.

| Throughput | X10 | X50 | X90 | X100 |
|---|---|---|---|---|
| 240 kg/h | 1.5 $\mu$m | 4.1 $\mu$m | 10.5 $\mu$m | 30.5 $\mu$m |

Viscosity: 83 mPa.s

**Example 7 Magaldrate R/magaldrate LD 1:1 about 20% MS50**

Grinding in an annular gap bead mill CoBall MS 50 mill
Motor 45 KW; grinding gap 7 mm; sieve 0.3 mm, separation gap 0.3 mm
Beads: glass 0.75 mm-1.0 mm,
Bead filling: see table, throughput: see table
Circumferential speed: 12.8 m/s
Amperage: average 30 A
Product temperature in: about 21°; product temperature out: average 20°C

Magaldrate powder R 70 kg and magaldrate powder LD 70 kg are dispersed with 460 kg of demineralized water. The dispersion is conveyed into the mill by means of a pump (NL30A) and Is ground. The concentration of anhydrous magaldrate is about 20% (w/w).

| Sample | Bead filling | Throughput (kg/h) | Viscosity MPa.s | Particles (Nm) X10/X50/X90/X100 |
|---|---|---|---|---|
| 1 | 55% | 600 | 73 | 1.5/4.2/12.3/43.5 |
| ½ (15 min run) | 55% | 600 | 66 | 1.5/4.1/11.8/36.5 |
| 2 | 60% | 600 | 27 | 1.7/7.0/27.4/61.5 |
| 3 | 45% | 600 | 32 | 1.7/4.9/12.2/30.5 |
| 4 | 50% | 640 | 53 | 1.6/4.4/10.7/25.5 |
| 5 | 50% | 528 | 64 | 1.6/4.2/10.2/25.5 |
| 6 | 55% | 440 | 122 | 1.4/3.7/9.1/25.5 |
| 7 | 55% | 650 | 73 | 1.5/4.1/10.8/30.5 |
| 8 | 55% | 500 | 107 | 1.5/3.8/9.1/21.5 |
| 9 (after stopping) | 55% | 500 | 93 | 1.5/4.0/10.6/36.5 |
| Commercial product | | | 67 | 2.2/5.0/10.0/36.5 |

### Example 8 Magaldrate R about 30% MS32

Grinding in an annular gap bead mill CoBall MS 32 mill
Motor 22 KW; grinding gap 7 mm; sieve 0.2 mm, separation gap 0.1 mm
Beads of zirconium silicate 0.8 mm-1.0 mm,
Bead filling 60%; throughput see table
Circumferential speed: 12.6 mls
Amperage: 25A
Product temperature in: 21°; product temperature out: 25°C

Magaldrate powder R 54.4 kg is dispersed with 100 kg of demineralized water. The concentration of anhydrous magaldrate is about 30%. To preserve the crude gel, 4.19 g of silver sulfate are added. The dispersion is conveyed into the mill by means of a pump (NL 15A) and is ground.

| Sample | Viscosity MPa.s | X10($\mu$m) | X50 ($\mu$m) | X90 ($\mu$m) | X100 ($\mu$m) |
|---|---|---|---|---|---|
| Un-ground | | 2 | 5 | 11 | 37 |
| 250 kg/h | 337 | 1.6 | 3.7 | 8.1 | 26 |
| 325 kg/h | 246 | 1.6 | 3.8 | 8.2 | 26 |

### Example 9 Magaldrate R/magaldrate LD 2:1 about 30% MS32

Grinding in an annular gap bead mill CoBaA MS 32 mill
Motor 22 KW; grinding gap 7 mm; sleve 0.2 mm, separation gap 0.1 mm
Beads of zirconium silicate 0.8 mm-1.0 mm,
Bead filling 60%; throughput see table
Circumferential speed: 12.6 m/s
Amperage: 25A
Product temperature in: 21°; product temperature out: 24°C

Magaldrate powder R 33 kg and magaldrate powder LD 16 kg are dispersed with 90 kg of demineralized water. The concentration of anhydrous magaldrate is about 30% (w/w). To preserve the crude gel. 3.766 g of silver sulfate are added. The dispersion is conveyed into the mill by means of a pump (NL 15A) and is ground.

| Sample | Viscosity MPa.s | X10 ($\mu$m) | X50 ($\mu$m) | X90 ($\mu$m) | X100 ($\mu$m) |
|---|---|---|---|---|---|
| 460 kg/h | 265 | 1.62 | 3.78 | 8.66 | 30.5 |
| 350 kg/h | 359 | 1.60 | 3.71 | 8.36 | 30.6 |

**Claims**

1. A process for the production of a rehydrated magaldrate preparation from magaldrate powder comprising the step of grinding of an aqueous suspension of magaldrate powder in a suitable mill.

2. The process as claimed in claim 1, wherein the rehydration of magaldrate powder requires no addition of excipients.

3. The process as claimed in claim 1, in which no organic or inorganic colloids are added.

4. The process as claimed in claim 1 or 3, wherein the aqueous suspension of magaldrate powder is obtained by adding magaldrate powder to water with stirring.

**5.** The process as claimed in claim 4, wherein a preservative is added.

**6.** The process as claimed in claim 5, wherein the preservative is silver sulfate.

**7.** The process as claimed in claim 1 or 3, where an aqueous suspension with 10 to 40% (w/w) magaldrate powder is employed.

**8.** The process as claimed in claim 7, where an aqueous suspension with 18 to 22% (w/w) magaldrate powder is employed.

**9.** The process as claimed in claim 1 or 3, where the mill is an annular gap bead mill.

**10.** A process for the production of liquid suspensions comprising magaldrate for use as pharmaceutical comprising the steps of

a. producing a rehydrated magaldrate preparation from magaldrate powder by grinding of an aqueous suspension of magaldrate powder in a suitable mill and
b. mixing of the rehydrated magaldrate preparation obtained under a) with at least one additive or a suitable solution of an additive, where the additive is selected from the group of flavorings, flocculation-preventing agents, thickeners, preservatives and sweeteners.

**11.** A process according to claim 10, where in step a. no organic or inorganic colloids are added.


**Patentansprüche**

**1.** Verfahren zur Herstellung rehydratisierter Magaldratzubereitungen aus Magaldratpulver umfassend den Schritt des Mahlens einer wässerigen Suspension von Magaldratpulver in einer geeigneten Mühle.

**2.** Verfahren nach Anspruch 1, wobei für die Rehydratisierung von Magaldratpulver keine Zugabe von Hilfsstoffen erforderlich ist.

**3.** Verfahren nach Anspruch 1, wobei keine organischen oder anorganischen Kolloide zugegeben werden.

**4.** Verfahren nach Anspruch 1 oder 3, wobei die wässrige Suspension von Magaldratpulver durch Zugabe von Magaldratpulver zu Wasser unter Rühren erhalten wird.

**5.** Verfahren nach Anspruch 4, wobei ein Konservierungsmittel zugegeben wird.

**6.** Verfahren nach Anspruch 5, wobei es sich bei dem Konservierungsmittel um Silbersulfat handelt.

**7.** Verfahren nach Anspruch 1 oder 3, wobei wässerige Suspensionen mit 10 bis 40% (w/w) Magaldratpulver eingesetzt werden.

**8.** Verfahren nach Anspruch 7, wobei wässrige Suspensionen mit 18 bis 22% (w/w) Magaldratpulver eingesetzt werden.

**9.** Verfahren nach Anspruch 1 oder 3, wobei es sich bei der Mühle um eine Ringspaltkugelmühle handelt.

**10.** Verfahren zur Herstellung flüssiger Suspensionen enthaltend Magaldrat zur Verwendung als Arzneimittel umfassend die Schritte

a) Herstellen rehydratisierter Magaldratzubereitungen aus Magaldratpulver durch Mahlen von wässrigen Suspensionen von Magaldratpulver in einer geeigneten Mühle und
b) Mischen der unter a) erhaltenen rehydratisierten Magaldratzubereitung mit mindestens einem Zusatzstoff oder einer geeigneten Lösung eines Zusatzstoffes, wobei der Zusatzstoff ausgewählt ist aus der Gruppe der Geschmacksmittel, die Ausflockung verhindernde Mittel, Verdickungsmittel, Konservierungsmittel und Süßstoffe.

**11.** Verfahren nach Anspruch 10, wobei in Schritt a. keine organischen oder anorganischen Kolloide zugegeben werden.

**Revendications**

**1.** Procédé de production d'une préparation de magaldrate réhydratée à partir de poudre de magaldrate, comprenant l'étape consistant à broyer une suspension aqueuse de poudre de magaldrate dans un broyeur approprié.

**2.** Procédé selon la revendication 1, selon lequel la réhydratation de la poudre de magaldrate ne nécessite aucun ajout d'excipients.

**3.** Procédé selon la revendication 1, selon lequel aucun colloïde organique ou minéral n'est n'ajouté.

**4.** Procédé selon la revendication 1 ou 3, selon lequel la suspension aqueuse de poudre de magaldrate est obtenue par l'ajout de poudre de magaldrate dans de l'eau avec agitation.

**5.** Procédé selon la revendication 4, selon lequel un conservateur est ajouté.

**6.** Procédé selon la revendication 5, selon lequel le conservateur est le sulfate d'argent.

**7.** Procédé selon la revendication 1 ou 3, selon lequel une suspension aqueuse comprenant 10 à 40 % (p/p) de poudre de magaldrate est utilisée.

**8.** Procédé selon la revendication 7, selon lequel une suspension aqueuse comprenant 18 à 22 % (p/p) de poudre de magaldrate est utilisée.

**9.** Procédé selon la revendication 1 ou 3, selon lequel le broyeur est un broyeur à billes à espace annulaire.

**10.** Procédé de production de suspensions liquides comprenant du magaldrate destinées à une utilisation en tant que produit pharmaceutique, comprenant les étapes consistant à :

a. produire une préparation de magaldrate réhydratée à partir de poudre de magaldrate en broyant une suspension aqueuse de poudre de magaldrate dans un broyeur approprié et
b. mélanger la préparation de magaldrate réhydratée obtenue en a. avec au moins un additif ou une solution appropriée d'un additif, l'additif étant choisi dans le groupe constitué des agents aromatisants, des agents anti-floculation, des épaississants, des conservateurs et des édulcorants.

**11.** Procédé selon la revendication 10, selon lequel, dans l'étape a., aucun colloïde organique ou minéral n'est ajouté.

**EP 1 351 887 B1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2923660 A

- DE 2749789